# EUROPÄISCHE PATENTSCHRIFT

(11) **EP 1 148 826 B1**
(45) Veröffentlichungstag und Bekanntmachung des Hinweises auf die Patenterteilung: **16.04.2003**
(21) Anmeldenummer: 00901511.6
(22) Anmeldetag: 07.01.2000
(51) Int. Cl.: A61B 17/16, A61F 2/36

(54) **HOHLRASPEL ZUR PRÄPARATION EINES RÖHRENKNOCHENS**
HOLLOW RASP FOR PREPARING A TUBULAR BONE
RAPE CREUSE POUR PREPARATION D'UN OS TUBULAIRE

(30) Priorität: 01.02.1999 DE 19903683
(43) Veröffentlichungstag der Anmeldung: 31.10.2001
(73) Patentinhaber: ESKA Implants GmbH & Co., 23556 Lübeck (DE)
(72) Erfinder: GRUNDEI, Hans, D-23558 Lübeck (DE)
(74) Vertreter: Weiss, Christian, Dipl.-Ing.
(86) Internationale Anmeldenummer: EP0000066
(87) Internationale Veröffentlichungsnummer: WO00045715

(56) Entgegenhaltungen:
- EP-A- 0 634 145
- WO-A-98/26725
- DE-A- 3 907 256
- DE-C- 19 543 530

## Beschreibung

Die Erfindung betrifft eine Hohlraspel zur Präparation eines Röhrenknochens für den Einsatz einer stielförmigen Endoprothese.

Vor der Implantation einer stielförmigen Endoprothese in einen Röhrenknochen, beispielsweise in den menschlichen Femur, wird dieser an seinem gelenkseitigen Ende reseziert und eine Ausfräsung der Spongiosa und Entfernung von Knochenmark vorgenommen. In den ausgefrästen Raum kann sodann der Stielteil der Endoprothese eingebracht werden, wo er zementlos oder unter Anwendung von Zement fixiert wird. Ein typisches Beispiel für eine derartige stielförmige Endoprothese ist ein Hüftstiel eines künstlichen Hüftgelenkes. Hohlraspeln sind im Stand der Technik bekannt. So sei hingewiesen auf die DE-U-9212906 und auf die DE-A-4323873 bzw. EP-A-0 634 145.

Die Hohlraspel gemäß der letztgenannten Druckschrift unterscheidet sich von der Hohlraspel gemäß der erstgenannten Druckschrift im wesentlichen dadurch, daß dort das Hohlrohr medial einen Längsschlitz aufweist. Hierdurch wird eine bessere Ausfräswirkung als bei der Raspel gemäß der erstgenannten Druckschrift erzielt. Darüber hinaus wird bei ihrer Anwendung das Risiko von Thrombosen und/oder Embolien erheblich reduziert, die auf eine vermehrte Knochenmarksfettausschüttung zurückgehen, die zu Fettembolien beim Patienten führen können.

Die Hohlschaftraspel gemäß der letztgenannten Druckschrift besteht im wesentlichen aus einem Federstahl mit einem Schabeprofil, das so ausgebildet ist, daß es nur in einer Bewegungsrichtung der Raspel wirkt. Abräummaterial wird beim Ausfräsen in das Innere des Hohlrohres gedrängt.

In der Praxis hat sich gezeigt, daß das im Stand der Technik verwendete Schabeprofil nicht ausreichend scharf ist, um ein rasches Ausräumen des Röhrenknochens zu erzielen.

Vor diesem Hintergrund ist es nun die Aufgabe der vorliegenden Erfindung, eine Hohlraspel der eingangs genannten Art vorzuschlagen, welche eine mehrfach erhöhte Raspelwirkung gegenüber den Hohlraspeln im Stande der Technik hat.

Gelöst wird die Aufgabe durch die Hohlraspel mit den Merkmalen des Anspruchs 1. Weitere vorteilhafte Ausbildungen ergeben sich aus den Unteransprüchen.

Demgemäß ist vorgesehen, daß die Hohlraspel, an die im übrigen proximal ein Handhabungsinstrument ankoppelbar ist, aus einem metallischen Gitternetzwerk besteht, das zumindest an seinen Knotenpunkten mit metallischen Raspelelementen bestückt ist, die integraler Bestandteil vom das Gitternetzwerk bildenden Material sind.

Insbesondere ist die erfindungsgemäße Hohlraspel mit Hilfe eines feingießtechnischen Wachsausschmelzverfahren hergestellt, so daß die Raspelelemente auch bei noch so hoher Belastung mit dem Gitternetzwerk verbunden bleiben. Die Raspelelemente wirken in allen Bewegungsrichtungen.

Bei rotationssymmetrischer Ausbildung der Hohlraspel ist es im übrigen möglich, die Hohlraspel mit dem Handhabungsinstrument in eine entsprechende motorisch angetriebene Maschine zu setzen und die Hohlraspel als motorisch betriebene Fräse zu verwenden. Die integral mit dem Gitternetzwerk ausgebildeten Raspelelemente halten diesen hohen Belastungen ohne weiteres stand.

Das während des Fräsvorganges abgeschabte Knochenmark und die abgeschabte Spongiosa werden in das Innere des Gitternetzwerkes gedrängt, so daß eine Embolie und/oder Thrombosegefahr jedenfalls vermieden wird.

Zum kontinuierlichen Raspeln oder Fräsen ist es vorteilhaft, wenn an die Hohlraspel proximal eine Absaugeinheit anschließbar ist, um die abgefrästen oder abgeraspelten Bestandteile absaugen zu können, wie per se an sich bekannt.

In besonders bevorzugter Ausführungsform werden die Raspelelemente gebildet aus einem Grundkörper mit wenigstens drei von diesem abragenden Zapfen. Diese Ausführung bietet eine bislang unbekannte Aggressivität einer Knochenraspel.

Gemäß einem besonderen Aspekt kann die Hohlraspel selbst als Implantat im ausgefrästen Knochenmarksraum verbleiben. Mit anderen Worten kann die Hohlraspel zunächst als solche eingesetzt werden und dann aber in dem von ihr geschaffenen Hohlraum verbleiben. Die Aggressivität der Oberfläche und ihr Hohlraum gestatten es, daß sich Knochenmaterial relativ rasch in den Hohlraum einorganisiert und die Raspelelemente umwächst. Besonders vorteilhaft ist dann die Möglichkeit, proximal das Handhabungsinstrument von der Hohlraspel abzukoppeln und beispielsweise mittels eines Doppelkonus mit einer Gelenkkugel zu versehen, woraufhin aus der Hohlraspel ein Stielteil eines künstlichen Hüftgelenkes geworden ist.

Die Erfindung wird anhand eines Ausführungsbeispieles näher erläutert. Hierbei zeigt:
- Figur 1: die schematische Ansicht einer Hohlraspel mit angekoppeltem Handhabungsinstrument sowie ein vergrößert dargestelltes Raspelelement, und
- Figur 2: die Möglichkeit, nach Ausübung der Raspelfunktion die Hohlraspel umzufunktionieren zu einem Implantat.

Nachfolgend sind dieselben Bezugszeichen für die gleichen Teile verwendet.

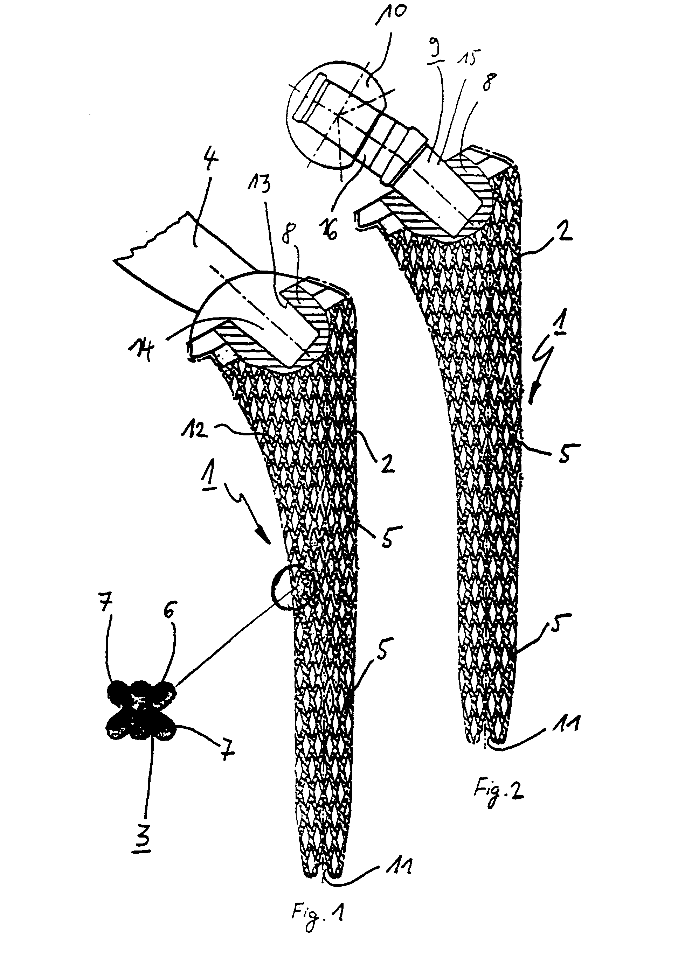

Figur 1 zeigt beispielhaft eine Hohlraspel 1 in etwa in der Form eines Stielteils eines künstlichen Hüftgelenkes. Die Hohlraspel 1 besteht im wesentlichen aus einem metallischen Gitternetzwerk 2, mit entsprechenden Öffnungen, durch welche das Abräummaterial in das Innere der Hohlraspel 1 gedrängt wird. Aus diesem Grunde ist am distalen Ende auch eine Öffnung 11 vorgesehen, durch welche Spongiose und/oder Knochenmark beim Vortrieb der Hohlraspel 1 in den Röhrenknochen in das Innere der Hohlraspel 1 gedrängt wird. Auf den Knotenpunkten 5 des Gitternetzwerkes 2 sowie auf den dazwischen gelegenen Stegen 12 befinden sich Raspelelemente 3, die vorliegen sechs von einem Grundkörper 6 abragende Zapfen 7 aufweisen. Es sind die Zapfen 7, welche der Hohlraspel 1 die enorme Aggressivität verleihen.

Proximalseitig ist ein Adapterstück 8 vorgesehen mit vorliegend beispielhaft dargestellter Steckhülse 13 für einen Steckkonus 14 eines Handhabungsinstrumentes 4, welches mit dem proximalen Teil der Hohlraspel 1 koppelbar ist.

In Figur 2 ist dieselbe Hohlraspel 1 dargestellt, allerdings wie sie bereits umfunktioniert ist, nachdem mit ihr in dem betroffenen Röhrenknochen ein Freiraum für das Implantat, welches vorliegend durch die Raspel 1 selbst gebildet wird, geschaffen worden ist.

Vorliegend ist der Steckkonus 14 des Handhabungsinstrumentes 4 aus Figur 1 ersetzt worden durch einen Doppelkonus 9 mit zwei Steckkonen 15 und 16. Auf den Steckkonus 16 ist sodann eine künstliche Gelenkkugel 10 eines künstlichen Gelenkes gesetzt worden. Die Hohlraspel 1 bildet somit ein vollständiges Implantat in Form eines Stielteils eines künstlichen Hüftgelenkes.

## Patentansprüche

1. Hohlraspel (1) zur Präparation eines Röhrenknochens für den Einsatz einer stielförmigen Endoprothese, an die proximal ein Handhabungsinstrument (4) ankoppelbar ist,
**dadurch gekennzeichnet, daß**
sie aus einem metallischen Gitternetzwerk (2) besteht, das zumindest an seinen Knotenpunkten (5) mit metallischen Raspelelementen (3) bestückt ist, die integraler Bestandteil vom das Gitternetzwerk (2) bildenden Material sind.

2. Hohlraspel nach Anspruch 1, bei der die Raspelelemente (3) gebildet sind aus einem Grundkörper (6) mit wenigstens drei von diesen abgragenden Zapfen (7).

3. Hohlraspel nach Anspruch 1 oder 2, bei dem proximal eine Absaugeinheit anschließbar ist.

4. Implantat für einen Röhrenknochen nach Ausräumung des Markraumes, das die Merkmale der Hohlraspel nach einem der Ansprüche 1 bis 3 aufweist.

## Claims

1. Hollow rasp (1) for preparation of a tubular bone for the insertion of a stem-like endoprosthesis, to which a manipulating instrument (4) can be coupled proximally, **characterised in that** it consists of a metallic lattice network (2), which at least at its points of intersection (5) is fitted with metallic rasp elements (3) which are an integral constituent of the material forming the lattice network (2).

2. Hollow rasp according to claim 1, in which the rasp elements (3) are formed from a base body (6) having at least three of these projecting pegs (7).

3. Hollow rasp according to claim 1 or 2, in which a suction unit can be connected proximally.

4. Implant for a tubular bone after clearing the medullary space, which has the features of the hollow rasp according to one of claims 1 to 3.

## Revendications

1. Râpe creuse (1) destinée à la préparation d'un os long pour l'implantation d'une endoprothèse en forme de tige, dont l'extrémité proximale peut être assemblée à un instrument de manipulation (4),
**caractérisée en ce qu'**
elle est formée par un treillis (2) métallique, qui comporte au moins au niveau de ses points d'intersection (5) des éléments de râpage (3) métalliques, qui font partie intégrante de la matière formant le treillis (2).

2. Râpe creuse selon la revendication 1, dans laquelle les éléments de râpage (3) sont formés par un corps de base (6) muni d'au moins trois tétons (7) en saillie sur celui-ci.

3. Râpe creuse selon la revendication 1 ou 2, dans laquelle une unité d'aspiration peut être raccordée à l'extrémité proximale.

4. Implant pour un os long après débourrage de la cavité médullaire, qui comprend les caractéristiques de la râpe creuse selon l'une des revendications 1 à 3.
